(19) Europäisches Patentamt
European Patent Office
Office européen des brevets

(11) Veröffentlichungsnummer: **0 280 165**
**B1**

(12) # EUROPÄISCHE PATENTSCHRIFT

(45) Veröffentlichungstag der Patentschrift: **01.08.90**

(51) Int. Cl.$^5$: **C07C 309/12**

(21) Anmeldenummer: **88102259.4**

(22) Anmeldetag: **17.02.88**

(54) **Verfahren zur Herstellung von Acyloxialkansulfonaten.**

(30) Priorität: **26.02.87 DE 3706230**

(43) Veröffentlichungstag der Anmeldung: **31.08.88 Patentblatt 88/35**

(45) Bekanntmachung des Hinweises auf die Patenterteilung: **01.08.90 Patentblatt 90/31**

(84) Benannte Vertragsstaaten: **AT BE CH DE ES FR GB IT LI NL SE**

(56) Entgegenhaltungen:
**EP-A- 0 182 017**
**US-A- 1 881 172**

(73) Patentinhaber: **HOECHST AKTIENGESELLSCHAFT, Postfach 80 03 20, D-6230 Frankfurt am Main 80(DE)**

(72) Erfinder: **Reinhardt, Gerd, Dr., Freiherr-vom-Stein-Strasse 37, D-6233 Kelkheim (Taunus)(DE)**
Erfinder: **Grabley, Fritz-Feo, Dr., Hölderlinstrasse 7, D-6240 Königstein/Taunus(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Acyloxialkansulfonsäuren und deren Salze sind seit langem bekannte Verbindungen mit oberflächenaktiven Eigenschaften.

Zu ihrer Herstellung sind bereits eine Reihe von Verfahren beschrieben. Dabei ist zu unterscheiden zwischen Reaktionen von Hydroxialkansulfonaten, insbesondere Na-Isethionaten, mit Fettsäuren (direkte Verfahren) oder mit deren Derivaten (indirekte Verfahren).

Bei der Direktkondensation wird Na-Isethionat mit einem Überschuß an Fettsäure, meist in Gegenwart eines Veresterungskatalysators, unter Abdestillation des Reaktionswassers umgesetzt. Als Katalysatoren sind u.a. Zinkoxid (US 3,320,292), Zinksalze organischer Säuren (US 4,405,526), phosphorhaltige Säuren (US 3,004,049) oder Borsäure (US 2,923,724) beschrieben. Bei diesen Reaktionen sind jedoch Veresterungstemperaturen von 220-250°C und lange Reaktionszeiten notwendig um eine ausreichende Umsetzung zu erreichen. Unter diesen Reaktionsbedingungen kommt es in vielen Fällen zu Produktschädigungen, z.B. Farbveränderungen.

Reaktionen mit aktivierten Säurederivaten weisen diese Nachteile nicht auf, jedoch sind diese Verfahren kostenintensiver, da ein zusätzlicher Reaktionsschritt zur Herstellung der Säurederivate erforderlich ist. Umsetzungen mit Säurechloriden (US 1,881,172, US 2,821,535), gemischten Borsäure-Fettsäureanhydriden (DAS 1 081 882) und Fettsäureisopropenylestern (US 3 745 181) sind bereits beschrieben. Davon hat insbesondere die Umsetzung der Fettsäurechloride industrielle Bedeutung erlangt. Dabei wird meist mit einem Überschuß an Na-Isethionat gearbeitet, um das Säurechlorid möglichst vollständig umzusetzen. Dennoch verbleiben im Endprodukt 1-3 % Natriumchlorid, die in einigen Anwendungsgebieten unerwünscht sind.

Acyloxiisethionate werden zusammen mit freier Fettsäure als Grundstoffe zur Herstellung von Syndet-Seifen eingesetzt (US 2 894 912, US 3 879 309, EP-A 176 330). So wird in US 4 180 470 für die Zusammensetzung eines verbesserten Seifen-Stückes u.a. angegeben: 30-70% Na-Acyloxialkansulfonat, 10-40 % freie Fettsäure, 1-7 % Na-Isethionat und 0,1-1 % Natriumchlorid.

Aufgabe der vorliegenden Erfindung ist, ein auch im großtechnischen Maßstab leicht durchführbares Verfahren zur Herstellung von Alkali- oder Erdalkalimetallsalzen von Acyloxialkansulfonsäuren aufzuzeigen, bei dem ein Natriumchlorid-armes bzw. -freies Produkt erhalten wird, das zudem freie Fettsäure enthält und somit ohne weitere Aufarbeitung zur Herstellung von Syndet-Seifen eingesetzt werden kann.

Gegenstand der Erfindung ist daher ein Verfahren zur Herstellung von Acyloxialkansulfonaten der Formel

$$R - \overset{\displaystyle O}{\overset{\|}{C}} - O - A - SO_3M$$

wobei R $C_7$-$C_{21}$-Alkyl oder $C_7$-$C_{21}$-Alkenyl, A $C_2$-$C_4$-Alkylen 2 bis 4 und M ein Alkali- oder Erdalkalimetall-Atom bedeuten, indem man Carbonsäurechloride der allgemeinen Formel R-COCl, wobei R die zuvor angegebene Bedeutung hat, mit einem Hydroxialkansulfonat der Formel HO-A-$SO_3$M, wobei A $C_2$-$C_4$-Alkylen und M ein Alkali- oder Erdalkalimetall ist, in Gegenwart einer Carbonsäure umsetzt.

Durch die Mitverwendung freier Säure als Lösemittel und Konsistenzregler kommt es zu einer besseren Durchmischung und damit zu hohen Umsetzungsgraden, ohne daß mit einem Überschuß an Fettsäurechlorid oder Hydroxialkansulfonat gearbeitet werden muß. Zudem kann das entstehende Chlorwasserstoffgas leichter aus dem Reaktionsgemisch entweichen, so daß ein chloridfreies oder zumindest chloridarmes Produkt erhalten wird, welches die Bedingungen als Ausgangsmaterial zur Herstellung von Syndet-Seifen erfüllt.

Das vorliegende Verfahren wird in der Weise durchgeführt, daß man ein Gemisch aus einem Mol Hydroxialkansulfonat mit 0,5-1,5 Mol, vorzugsweise 0,8-1,2 Mol, eines Carbonsäurechlorids und 0,1-1,0 Mol, vorzugsweise 0,4-0,7 Mol, einer Carbonsäure vermischt und die Mischung, ggf. unter Inertgasatmosphäre, auf 90-200°C, vorzugsweise 140-180°C, erhitzt. Um ein Aufschäumen des Reaktionsgemisches beim Einsetzen der Reaktion zu vermeiden, wird in einer besonderen Ausführungsform dieses Verfahren eine Mischung aus Hydroxialkansulfonat und Säure vorgelegt und dazu bei 90-200°C, vorzugsweise 100-130°C, das Säurechlorid hinzugetropft. In einer weiteren bevorzugten Ausführungsform werden äquimolare Mischungen von Säure und Säurechlorid zunächst auf 150-180°C erhitzt, wobei sich unter HCl-Entwicklung ein Gemisch von Säureanhydriden bildet. Nach beendeter Gasentwicklung wird das Reaktionsgemisch auf 120°C abgekühlt, das Hydroxialkansulfonat zugegeben und anschließend das restliche Säurechlorid zugetropft. Zur Vervollständigung der Reaktion und um den entstehenden Chlorwasserstoff möglichst vollständig zu entfernen, wird das Reaktionsgemisch anschließend auf 120-200°C, vorzugsweise 140-180°C erhitzt. Die Reaktionszeit beträgt 30 min - 10 h, vorzugsweise 2-5 h. Das anfangs dünnflüssige Reaktionsgemisch wird bei fortschreitender Umsetzung zähflüssig, bleibt jedoch bei Temperaturen oberhalb 160°C gut rührbar. Es ist auch möglich, die Reaktion in einem Kneter o.ä. durchzuführen.

Während oder nach der Reaktion kann die zugesetzte Carbonsäure ganz oder teilweise aus dem Reaktionsgemisch durch Destillation unter reduziertem Druck entfernt werden. Weiterhin ist es möglich, die überschüssige Carbonsäure durch Lösemittelextraktion z.B. mit Paraffinen oder Alkoholen wie i-Propanol zu entfernen, um so zu Wirkstoffgehalten von mehr als 90 % zu gelangen.

Das Endprodukt ist pH-neutral oder schwach sauer und kann, falls erforderlich, durch Zugabe von Alkali- oder Erdalkalicarbonaten oder -hydrogencarbonaten im pH-Wert korrigiert werden.

Typische Hydroxialkansulfonate, die eingesetzt werden können, sind Alkali- oder Erdalkalimetallsalze, insbesondere Natriumsalze von Monohydroxialkansulfonsäuren mit 2 bis 4 Kohlenstoffatomen, insbesondere 2-Hydroxialkansulfonsäuren, z.B. 2-Hydroxiethansulfonsäure (Isethionsäure), 2-Hydroxipropansulfonsäure und 2-Hydroxibutansulfonsäure.

Typische Carbonsäuren, die eingesetzt werden können, sind alle geradkettigen oder verzweigten Carbonsäuren, insbesondere höhere Fettsäuren, gesättigt oder ungesättigt, mit 8 bis 22 Kohlenstoffatomen wie z.B. Octansäure, Laurinsäure, Palmitinsäure, Stearinsäure oder Mischungen davon wie z.B. Talgfettsäuren, Tallölfettsäuren oder Kokosfettsäuren. Die Carbonsäurechloride der Formel RCOCL können aus den zugrundeliegenden Säuren nach bekannten Methoden, wie z.B. Umsetzung mit Phosphortrichlorid hergestellt werden.

Unter dem Begriff WS-Gehalt ist in den nachfolgenden Beispielen der Anteil an waschaktiver Substanz im Produkt zu verstehen, der durch 2-Phasen-Titration nach EPTON (Nature 160, 759 (1947)) bestimmt wird.

**Beispiel 1**

148 Teile trockenes Na-Isethionat werden in 111 Teilen gehärteter Kokosfettsäure($C_{12}C_{18}$) aufgeschlämmt und unter Stickstoffatmosphäre auf 90°C erhitzt. Innerhalb 1 h werden 240,5 Teile gehärtetes Kokosfettsäurechlorid zugetropft. Es wird 2 h bei 120°C und 1 bei 180°C nachgerührt. Währenddessen entsteht aus der dünnflüssigen Suspension eine zähflüssige, aber gut rührbare Masse. Gegen Ende der Reaktion wird der pH-Wert durch Zugabe von Natriumcarbonat auf pH = 6,0 eingestellt. Nach dem Erkalten wird das spröde, weiße Produkt gepulvert. Der WS-Gehalt des Produktes beträgt 73,8 %, der Umsetzungsgrad 91,6 % bezogen auf eingesetztes Oxethansalz. Das Produkt enthält 0,3 % Natriumchlorid.

**Beispiel 2**

148 Teile Na-Isethionat werden in 106,5 Teilen gehärteter Kokosfettsäure($C_{12}$-$C_{18}$) aufgeschlämmt und unter Stickstoff auf 120°C erhitzt. Dazu werden dann 240,5 Teile Kokosfettsäurechlorid($C_{12}$-$C_{18}$) innerhalb 1 h zugetropft, anschließend wird das Reaktionsgemisch 3 h auf 160-170°C erhitzt. Während dieser Zeit entsteht aus dem dünnflüssigen ein zähflüssiges, aber noch gut rührbares Reaktionsgemisch. Nach dem Erkalten wird des Reaktionsgemisch zerkleinert. Man erhält 458 Teile weißes Pulver mit einem WS-Gehalt von 74 %. Der Umsetzungsgrad (bezogen auf Na-Isethionat) beträgt 96,4 %, der $Cl^{\ominus}$-Gehalt beträgt 0,15 %.

**Beispiel 3**

148 Teile Na-Isethionat werden in 106,5 Teilen gehärteter Kokosfettsäure($C_{12}$-$C_{18}$) aufgeschlämmt. Bei 125°C werden dann unter Stickstoffatmosphäre 252 Teile Kokosfettsäurechlorid innerhalb 45 min hinzugetropft. Das Reaktionsgemisch wird anschließend auf 175°C erhitzt. Der WS-Gehalt beträgt nach 2 h 65 %, nach 3 h 75,8 %. Der Umsetzungsgrad beträgt 98,7 %, der $Cl^{\ominus}$-Gehalt liegt unter 0,1%.

**Beispiel 4**

Es wird analog Beispiel 3 verfahren, jedoch wird nach einer Reaktionszeit von 2 h bei 175°C ein starker Stickstoffstrom durch das Gemisch geleitet, um Fettsäure aus dem Reaktionsgemisch zu entfernen. Nach 1 beträgt der WS-Gehalt 80,1 %, der Umsetzungsgrad 99 %. Chlorid ist nicht nachweisbar.

**Beispiel 5**

Es wird analog Beispiel 3 verfahren. Nach einer Reaktionszeit von 3 h bei 175°C wird das heiße Reaktionsgemisch in 800 Vol.-Teile i-Propanol gegeben und 1 h zum Rückfluß erhitzt. Nach dem Erkalten wird das kristalline Acylisethionat abfiltriert und getrocknet. Man erhält 370 Teile weißes Pulver mit einem WS-Gehalt von 94,5 %.

**Patentansprüche**

1. Verfahren zur Herstellung von oberflächenaktiven Acyloxialkansulfonaten der Formel

$$R - \overset{\overset{\Large O}{\|}}{C} - O - A - SO_3M$$

wobei R $C_7$-$C_{21}$-Alkyl oder $C_7$-$C_{21}$-Alkenyl, A $C_2$-$C_4$-Alkylen und M ein Alkali- oder Erdalkalimetall-Atom bedeuten, dadurch gekennzeichnet, daß man Carbonsäurechloride der allgemeinen Formel R-COCl, wobei R die zuvor angegebene Bedeutung hat, mit einem Hydroxialkansulfonat der Formel HO-A-$SO_3M$, wobei A $C_2$-$C_4$-Alkylen und M ein Alkali- oder Erdalkalimetall ist, in Gegenwart einer Carbonsäure umsetzt.

2. Verfahren nach Anspruch 1, dadurch gekennzeichnet, daß man die Reaktion bei einer Temperatur von 90-200°C durchführt.

3. Verfahren nach Anspruch 1, dadurch gekennzeichnet, daß zu einem Gemisch aus Hydroxialkansulfonaten und Carbonsäure bei Temperaturen zwischen 90 und 200°C das Carbonsäurechlorid hinzugetropft wird.

4. Verfahren nach Anspruch 1, dadurch gekennzeichnet, daß man ein Gemisch im Verhältnis 1 Mol Hydroxialkansulfonat, 0,5 bis 1,5 Mol Carbonsäurechlorid und 0,1 bis 1 Mol Carbonsäure umsetzt.

5. Verfahren nach Anspruch 1, dadurch gekennzeichnet, daß man als Carbonsäure eine $C_8$-$C_{22}$-Carbonsäure einsetzt.

6. Verwendung der nach einem der Ansprüche 1 bis 3 hergestellten Acyloxialkansulfonate als obeflächenaktive Verbindung in Syndet-Seifen.

**Claims**

1. A process for the preparation fo surface-active acyloxyalkanesulfonates of the formula

$$R-\overset{\overset{O}{\|}}{C}-O-A-SO_3M$$

in which R denotes $C_7$–$C_{21}$-alkyl or $C_7$–$C_{21}$-alkenyl, A denotes $C_2$–$C_4$-alkylene and M denotes an alkali metal or alkaline earth metal atom, which comprises reacting, in the presense of a carboxylic acid, carboxylic acid chlorides of the formula R–COCl in which R has the meaning indicated above with a hydroxyalkanesulfonate of the formula HO-A-$SO_3$M in which A is $C_2$–$C_4$-alkylene and is an alkali metal or alkaline earth metal.

2. The process as claimed in claim 1, wherein the reaction is carried out at a temperature of 90–200°C.

3. The process as claimed in claim 1, wherein the carboxylic acid chloride is added dropwise to a mixture of hydroxyalkanesulfonate and carboxylic acid at temperatures between 90 and 200°C.

4. The process as claimed in claim 1, wherein the mixture reacted is in the proportions of 1 mole of hydroxyalkanesulfonate, 0.5 to 1.5 moles of carboxylic acid chloride and 0.1 to 1 mole of carboxylic acid.

5. The process as claimed in claim 1, wherein the carboxylic acid employed is a $C_8$–$C_{22}$ carboxylic acid.

6. The use of the acyloxyalkanesulfonates prepared as claimed in claim 1 as the surface-active compound in syndet soaps.

## Revendications

1. Procédé pour préparer des acyloxy-alcane-sulfonates surfactifs qui répondent à la formule:

$$R-\overset{\overset{O}{\|}}{C}-O-A-SO_3M$$

dans laquelle
R représente un alkyle en $C_7$–$C_{21}$ ou un alcényle en $C_7$–$C_{21}$,
A représente un alkylène en $C_2$–$C_4$ et
M représente un atome de métal alcalin ou de métal alcalino-terreux,
procédé caractérisé en ce qu'on fait réagir des chlorures d'acides carboxyliques de formule générale R–COCl, dans lesquels R a la signification qui vient d'être indiquée, avec un hydroxy-alcane-sulfonate de formule HO-A-$SO_3$M, dans lequel A représente un alkylène en $C_2$–$C_4$ et M un métal alcalin ou alcalino-terreux, en présence d'un acide carboxylique.

2. Procédé selon la revendication 1, caractérisé en ce qu'on effectue la réaction à une température de 90 à 200°C.

3. Procédé selon la revendication 1, caractérisé en ce qu'on ajoute goutte à goutte le chlorure d'acide carboxylique à un mélange d'hydroxy-alcane-sulfonates et d'un acide carboxylique, à une température comprise entre 90 et 200°C.

4. Procédé selon la revendication 1, caractérisé en ce qu'on fait réagir, en proportion, 1 mol de l'hydroxy-alcane-sulfonate, de 0,5 à 1,5 mol du chlorure d'acide carboxylique et de 0,1 à 1 mol de l'acide carboxylique.

5. Procédé selon la revendication 1, caractérisé en ce qu'on utilise, comme acide carboxylique, un acide carboxylique contenant de 8 à 22 atomes de carbone.

6. Application des acyloxy-alcane-sulfonates préparés selon l'une quelconque des revendications 1 à 3, comme composés surfactifs dans des savons contenant des détergents synthétiques.